# EUROPÄISCHE PATENTANMELDUNG

(11) **EP 2 719 354 A2**
(43) Veröffentlichungstag der Anmeldung: **16.04.2014**
(21) Anmeldenummer: 13180909.7
(22) Anmeldetag: 19.08.2013
(51) Int. Cl.: A61B 19/00, A61B 17/00

(54) **Instrumentenhalterung**

(30) Priorität: 10.10.2012 DE 102012019852
(71) Anmelder: Gebr. Brasseler GmbH & Co. KG, 32657 Lemgo (DE)
(72) Erfinder: Krumsiek, Michael, 32657 Lemgo (DE); Tünker, Stefan, 32657 Lemgo (DE); Neumeyer, Dr. Stefan, 93458 Eschlkam (DE)
(74) Vertreter: Hoefer & Partner

(57) **Zusammenfassung**

Die Erfindung bezieht sich auf eine Instrumentenhalterung mit einem im Wesentlichen rohrförmigen Grundkörper 1, welcher einen Griffbereich 2 und einen Instrumentenhaltebereich 3 umfasst, wobei der Grundkörper 1 eine sich über seine gesamte Länge erstreckende Durchgangsausnehmung 4 aufweist und wobei der Instrumentenhaltebereich 3 mit einer stirnseitigen, zur Einführung eines Instruments ausgebildeten Einstecköffnung 5 versehen ist.

## Beschreibung

Die Erfindung bezieht sich auf eine Instrumentenhalterung, welche dazu dient, ein Dentalinstrument oder ein medizinisches Instrument zu halten und dessen Handhabung zu ermöglichen.

Auf dem Dentalgebiet bzw. dem Gebiet der Dentalmedizin ist es erforderlich, Instrumente zu handhaben, beispielsweise, um sie in ein Winkelstück oder eine ähnliche Antriebseinrichtung einzusetzen und aus dieser zu entnehmen. Die Instrumente, welche beispielsweise in Form von Sägen, Skalpellen oder spanenden Werkzeugen ausgebildet sein können, weisen dabei scharfe Schneiden auf, so dass es erforderlich ist, bei der Handhabung besondere Vorsicht walten zu lassen. Weiterhin sind die Instrumente vielfach sehr klein dimensioniert, so dass deren Handhabung mit OP-Handschuhen nicht ganz einfach ist. Eine weitere Thematik ergibt sich daraus, dass während einer Behandlung oder Operation die Instrumente häufiger gewechselt werden müssen, wodurch sich die Notwendigkeit einer einfachen und schnellen Handhabung ergibt.

Der Erfindung liegt die Aufgabe zugrunde, eine Instrumentenhalterung zu schaffen, welche den Anforderungen gerecht wird und eine sichere, einfache und reproduzierbare Halterung von unterschiedlichen Instrumenten ermöglicht.

Erfindungsgemäß wird die Aufgabe durch Merkmalskombination des Anspruchs 1 gelöst, die Unteransprüche zeigen weitere vorteilhafte Ausgestaltungen der Erfindung.

Erfindungsgemäß ist somit eine Instrumentenhalterung geschaffen, welche einen im Wesentlichen rohrförmigen Grundkörper aufweist. Der Grundkörper umfasst einen Griffbereich und einen Instrumentenhaltebereich. Weiterhin ist der Grundkörper mit einer sich über seine gesamte Länge erstreckenden Durchgangsausnehmung versehen. Weiterhin ist vorgesehen, dass der Instrumentenhaltebereich eine stirnseitige, zur Einführung eines Instruments ausgebildete Einstecköffnung aufweist. Somit ist es möglich, in die Instrumentenhalterung ein Dentalinstrument oder medizinisches Instrument einzustecken und dieses somit zu haltern.

Die erfindungsgemäße Instrumentenhalterung dient somit dazu, Dentalinstrumente oder medizinische Instrumente zu deren Handhabung zu halten. Somit ist es in einfacher Weise möglich, die Instrumente beispielsweise in eine Antriebsvorrichtung einzusetzen oder aus dieser zu entnehmen, ohne dass die Instrumente selbst direkt manuell gegriffen werden müssen. Die erfindungsgemäße Instrumentenhalterung dient somit dazu, ein direktes manuelles Greifen der Instrumente zu umgehen, indem diese in die Instrumentenhalterung eingesteckt und zusammen mit dieser gehandhabt werden. Somit ist kein direkter manueller Kontakt mit den Instrumenten erforderlich. Dies schließt das Verletzungsrisiko für den Arzt oder seine Assistenten aus. Weiterhin können auch sehr kleine Instrumente sicher gehandhabt werden, da die Instrumentenhalterung selbst ausreichend groß dimensioniert sein, um diese sicher und bequem greifen zu können. Die erfindungsgemäße Instrumentenhalterung stellt somit einen Multifunktionsgriff dar.

Die erfindungsgemäße Instrumentenhalterung kann weiterhin dazu dienen, einzelne Instrumente während deren gesamtem Verwendungszyklus zu handhaben. Somit können die Instrumente bereits in jeweils einer Instrumentenhalterung dem Zahnarzt oder Operateur bereitgestellt werden. Die Instrumente können somit im sterilen Zustand zusammen mit der Instrumentenhalterung angeliefert werden, so dass keine vorherige separate manuelle Handhabung erforderlich ist. Dabei kann es sich als besonders günstig erweisen, wenn die Instrumentenhalterung mit geeigneten Beschriftungen oder Markierungen versehen ist, beispielsweise auch farblich gestaltet ist, um dem Zahnarzt oder Operateur die Information zu geben, welches Instrument sich in der jeweiligen Instrumentenhalterung befindet. Dieser kann dann die Instrumentenhalterung zusammen mit dem Instrument in eine Antriebseinrichtung, beispielsweise ein Winkelstück, einsetzen. Nach dem Gebrauch des Instruments wird dieses dann durch die Instrumentenhalterung aufgenommen und aus dem Antriebsmechanismus entfernt. In besonders günstiger Ausgestaltung der Erfindung ist es auch möglich, die Instrumentenhalterung zusammen mit dem Instrument zu reinigen und dem Hygienekreislauf zuzuführen. Eine manuelle Berührung des Arbeitsbereichs des Instruments kann damit verhindert werden.

In besonders günstiger Ausgestaltung der Erfindung ist vorgesehen, dass der Instrumentenhaltebereich mit zumindest einer seitlichen Ausnehmung versehen ist. Durch diese Ausnehmung kann eine Sichtkontrolle erfolgen, ob sich in der Instrumentenhalterung ein Instrument befindet und welches Instrument durch die Instrumentenhalterung aufgenommen ist.

Um das Instrument verliersicher in der Instrumentenhalterung zu haltern, ist es vorteilhaft, wenn die Einstecköffnung mit zumindest einem elastischen Klemmelement versehen ist. Dieses Klemmelement kann beispielsweise in Form einer elastischen Zunge ausgebildet sein, welche gegen den Schaft eines Instruments in Anlage gebracht wird. Hierdurch wird verhindert, dass sich bei der Handhabung der Instrumentenhalterung das Instrument löst und aus dieser herausfällt. Bevorzugt sind im Bereich der Einstecköffnung mehrere derartige elastische Klemmelemente vorgesehen.

Um eine Reinigung der Instrumente zu verbessern, kann es vorteilhaft sein, wenn im Bereich der Einstecköffnung zumindest eine Spülausnehmung ausgebildet ist. Durch diese Spülausnehmung kann eine Reinigungsflüssigkeit durch den Innenbereich der Instrumentenhalterung durchgeleitet werden, um das Instrument selber zu säubern.

Der Griffbereich ist bevorzugterweise mit einer Griffmulde versehen, um auch unter erschwerten Handhabungsbedingungen, beispielsweise mit OP-Handschuhen, die Instrumentenhalterung sicher und zuverlässig greifen zu können.

Zwischen dem Griffbereich und dem Instrumentenhaltebereich ist in günstiger Weiterbildung der Erfindung ein ringwulstförmiger Zwischenbereich ausgebildet. Dieser dient auch zur Aufbringung axialer Kräfte, um das Instrument mittels der Instrumentenhalterung in einen Antriebsmechanismus (beispielsweise ein Winkelstück) einfügen bzw. entnehmen zu können. Zudem wird eine sichere Handhabung der Instrumentenhalterung selbst erzielt.

Der Griffbereich ist bevorzugterweise so ausgebildet, dass er zur Aufnahme der Instrumentenhalterung in einem Tray geeignet ist und/oder in ein Desinfektionsgerät oder eine zugehörige Hilfsvorrichtung einsteckbar ist.

Weiterhin kann es günstig sein, wenn der Instrumentenhaltebereich mit einer Verdrehsicherung versehen ist, so dass eine exakte Zuordnung des Instruments erfolgen kann.

Die erfindungsgemäße Instrumentenhalterung kann aus unterschiedlichen Werkstoffen gefertigt sein, beispielsweise aus medizinisch zugelassenen Kunststoffen, wie Peek oder LPC. Es ist jedoch auch möglich, die Instrumentenhalterung aus metallischen Werkstoffen, wie Titan, Titanlegierungen oder Edelstahl, zu fertigen.

Durch geeignete Wahl des Werkstoffs der Instrumentenhalterung ist es somit, wie oben stehend beschrieben, möglich, die Instrumentenhalterung zusammen mit einem Instrument in einen Thermodesinfektor einzubringen, um die Instrumentenhalterung zusammen mit dem Instrument zu reinigen oder zu desinfizieren. Dabei kann, wie erwähnt, eine Spül- und Reinigungsflüssigkeit durch die Instrumentenhalterung durchgeleitet werden.

Am freien Endbereich weist die erfindungsgemäße Instrumentenhalterung, angrenzend an die Einstecköffnung, bevorzugterweise zumindest einen Biegeschlitz auf. Hierdurch ist es möglich, dass der Zahnarzt während der Behandlung das Instrument mit der Instrumentenhalterung greift und biegen kann, so dass das Instrument der anatomischen Form des zu behandelnden Zahnes angepasst werden kann.

Im Folgenden wird die Erfindung anhand von Ausführungsbeispielen in Verbindung mit der Zeichnung beschrieben. Dabei zeigt:
- Fig. 1: eine perspektivische Ansicht eines ersten Ausführungsbeispiels einer erfindungsgemäßen Instrumentenhalterung mit eingesetztem Instrument,
- Fig. 2 und 3: axiale Schnittansichten des in Fig. 1 gezeigten Ausführungsbeispiels der Instrumentenhalterung,
- Fig. 4: eine perspektivische Ansicht, ähnlich Fig. 1, eines weiteren Ausführungsbeispiels, und
- Fig. 5: eine Schnittansicht des in Fig. 4 gezeigten Ausführungsbeispiels.

Bei den nachfolgenden Ausführungsbeispielen sind gleiche Teile mit gleichen Bezugsziffern versehen.

Die erfindungsgemäße Instrumentenhalterung umfasst gemäß einem ersten Ausführungsbeispiel einen im Wesentlichen rohrförmigen Grundkörper 1, welcher mit einem Griffbereich 2 und einem Instrumentenhaltebereich 3 versehen ist. Zwischen dem Griffbereich 2 und dem Instrumentenhaltebereich 3 ist ein ringwulstförmiger Zwischenbereich 10 ausgebildet, welcher einen vergrößerten Durchmesser aufweist. Der Griffbereich 3 ist mit einer Griffmulde 9 versehen.

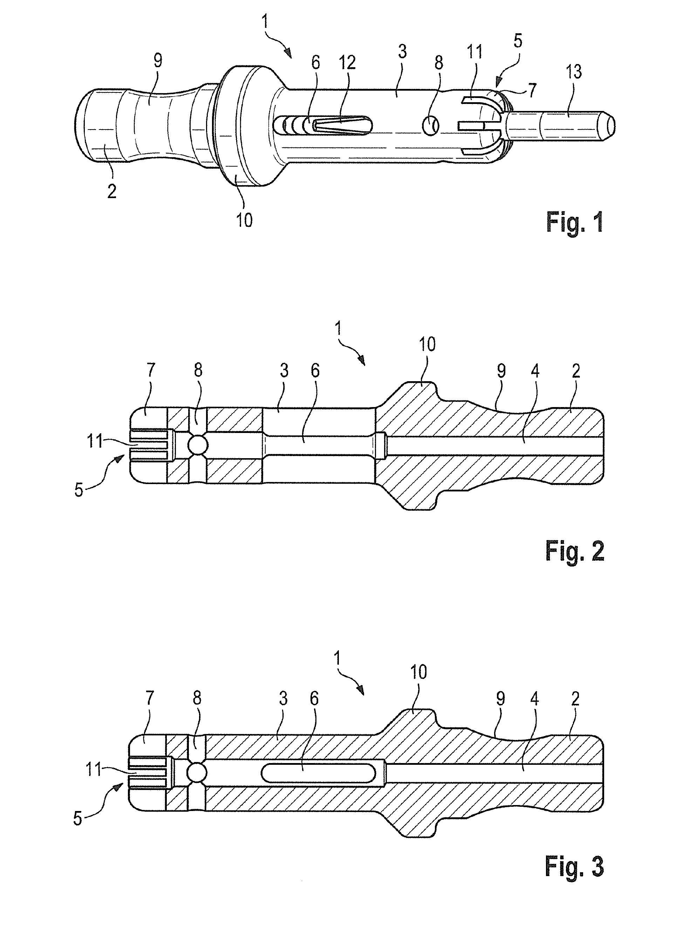

Durch die gesamte Länge der erfindungsgemäßen Instrumentenhalterung erstreckt sich eine Durchgangsausnehmung 4 (s. Fig. 2 und 3).

Am freien Ende des Instrumentenhaltebereichs 3 sind mehrere Klemmelemente 7 ausgebildet, welche in Form von elastischen Fingern ausgestaltet sind. Zwischen diesen Klemmelementen 10 sind Biegeschlitze 11 vorgesehen.

Der Instrumentenhaltebereich 3 weist seitliche Ausnehmungen 6 auf, welche als Sichtfenster dienen.

Wie in Figur 1 gezeigt, kann ein Dentalinstrument oder medizinisches Instrument durch eine stirnseitige Einstecköffnung 5 in die Durchgangsausnehmung 4 der Instrumentenhalterung eingesteckt werden. Ein Arbeitskopf 12 des Instruments wird dabei im eingesteckten Zustand so positioniert, dass er sich im Bereich der seitlichen Ausnehmungen 6 befindet. Durch diese ist es somit wie durch ein Sichtfenster möglich, den Arbeitskopf zu sehen, um zum einen dessen Lage zu kontrollieren und zum anderen festzustellen, ob das richtige Instrument in der Instrumentenhalterung angeordnet ist. Ein Schaft 13 des Instruments wird dabei durch die Klemmelemente 7 geklemmt und gehaltert, um ein unbeabsichtigtes Herausfallen des Instruments zu verhindern.

Die Figuren zeigen weiterhin im Bereich des Instrumentenhaltebereichs 3 zusätzliche Spülausnehmungen 8, durch welche eine Reinigungs- oder Spülflüssigkeit eingeleitet oder ausgeleitet werden kann.

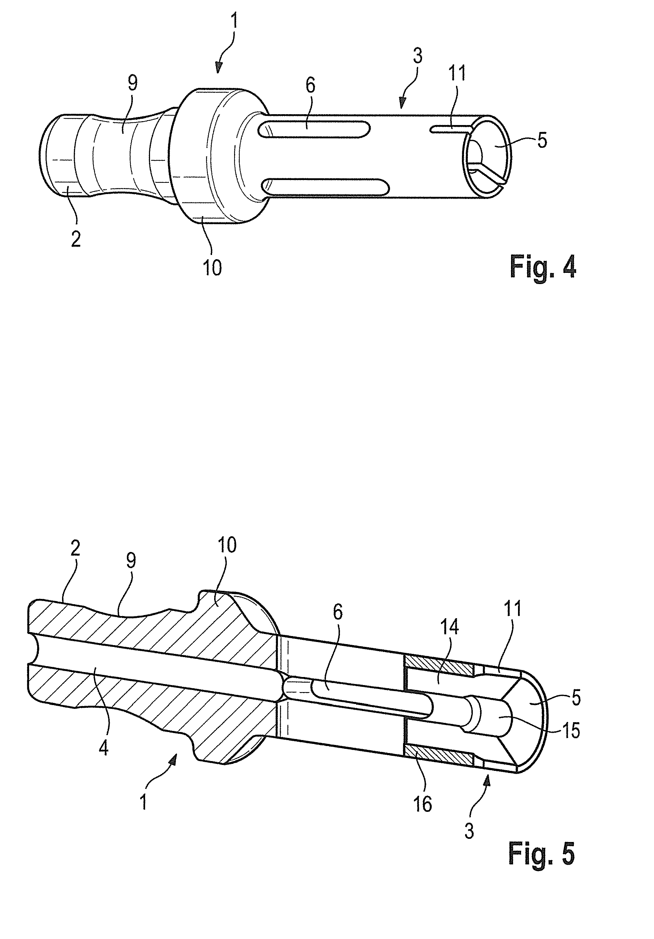

Die Fig. 4 und 5 zeigen ein weiteres Ausführungsbeispiel der Erfindung. Dieses unterscheidet sich von dem Ausführungsbeispiel der Fig. 1 bis 3 hinsichtlich der Ausgestaltung der Einstecköffnung. Diese ist trichterförmig ausgebildet, um ein Einführen eines Instruments zu erleichtern. Weiterhin sind angrenzend an die trichterförmige Einstecköffnung 5 zwei einander gegenüberliegende Biegeschlitze 11 vorgesehen, die, wie bei dem Ausführungsbeispiel der Fig. 1 bis 3, die Möglichkeit eröffnen, ein Instrument zu biegen.

Die Durchgangsausnehmung 4 erstreckt sich durch die gesamte Länge des Grundkörpers 1, analog der Ausgestaltung gemäß Fig. 1 bis 3. Im Instrumentenhaltebereich 3 ist die Durchgangsausnehmung 4 erweitert und bildet somit einen erweiterten Bereich 14 (siehe Fig. 5). Auch angrenzend an die trichterförmige Einstecköffnung 5 ist die Durchgangsausnehmung 4 mit einem größeren Durchmesser versehen, so wie dies durch das Bezugszeichen 15 dargestellt ist. Der erweiterte Bereich 14 ist in Verlängerung der Biegeschlitze 11 ausgebildet, so dass der Instrumentenhaltebereich 3, wie in Fig. 5 dargestellt, die Stege 16 umfasst und von diesen begrenzt wird.

### Bezugszeichenliste

- 1: Grundkörper
- 2: Griffbereich
- 3: Instrumentenhaltebereich
- 4: Durchgangsausnehmung
- 5: Einstecköffnung
- 6: seitliche Ausnehmung
- 7: Klemmelement
- 8: Spülausnehmung
- 9: Griffmulde
- 10: Zwischenbereich
- 11: Biegeschlitz
- 12: Arbeitskopf
- 13: Schaft
- 14: erweiterter Bereich
- 15: Durchmesserbereich
- 16: Steg

## Patentansprüche

1. Instrumentenhalterung mit einem im Wesentlichen rohrförmigen Grundkörper (1), welcher einen Griffbereich (2) und einen Instrumentenhaltebereich (3) umfasst, wobei der Grundkörper (1) eine sich über seine gesamte Länge erstreckende Durchgangsausnehmung (4) aufweist und wobei der Instrumentenhaltebereich (3) mit einer stirnseitigen, zur Einführung eines Instruments ausgebildeten Einstecköffnung (5) versehen ist.

2. Instrumentenhalterung nach Anspruch 1, **dadurch gekennzeichnet, dass** der Instrumentenhaltebereich (3) mit zumindest einer seitlichen Ausnehmung (6) versehen ist.

3. Instrumentenhalterung nach Anspruch 1 oder 2, **dadurch gekennzeichnet, dass** die Einstecköffnung (5) mit zumindest einem elastischen Klemmelement (7) versehen ist.

4. Instrumentenhalterung nach einem der Ansprüche 1 bis 3, **dadurch gekennzeichnet, dass** im Bereich der Einstecköffnung (5) zumindest eine Spülausnehmung (8) ausgebildet ist.

5. Instrumentenhalterung nach einem der Ansprüche 1 bis 4, **dadurch gekennzeichnet, dass** der Griffbereich (2) mit zumindest einer Griffmulde (9) versehen ist.

6. Instrumentenhalterung nach einem der Ansprüche 1 bis 5, **dadurch gekennzeichnet, dass** zwischen dem Griffbereich (2) und dem Instrumentenhaltebereich (3) ein ringwulstförmiger Zwischenbereich (10) ausgebildet ist.

7. Instrumentenhalterung nach einem der Ansprüche 1 bis 6, **dadurch gekennzeichnet, dass** der Griffbereich (2) zur Aufnahme der Instrumentenhalterung in einem Desinfektionsgerät ausgebildet ist.

8. Instrumentenhalterung nach einem der Ansprüche 1 bis 7, **dadurch gekennzeichnet, dass** der Instrumentenhaltebereich (3) mit einer Verdrehsicherung versehen ist.

9. Instrumentenhalterung nach einem der Ansprüche 1 bis 8, **dadurch gekennzeichnet, dass** im Bereich der Einstecköffnung (5) zumindest ein Biegeschlitz (11) vorgesehen ist.
